Europäisches Patentamt

European Patent Office

Office européen des brevets

(11) Publication number: **0 450 398 A2**

# EUROPEAN PATENT APPLICATION

(21) Application number: 91104299.2

(51) Int. Cl.⁵: **C08B 37/08, A61K 31/725**

(22) Date of filing: **20.03.91**

(30) Priority: **05.04.90 IT 1994890**

(43) Date of publication of application:
**09.10.91 Bulletin 91/41**

(84) Designated Contracting States:
**AT BE CH DE DK ES FR GB GR IT LI LU NL SE**

(71) Applicant: **BIOINDUSTRIA FARMACEUTICI S.p.A.**
**Via De Ambrosiis, 2-4-6**
**I-15067 Novi Ligure Alessandria(IT)**

(72) Inventor: **Federici, Giorgio**
**Via De Ambrosiis 2-4-6**
**I-15067 Novi Ligure, (Alessandria)(IT)**
Inventor: **Bertolotto, Rosa Maria**
**Via De Ambrosiis 2-4-6**
**I-15067 Novi Ligure, (Alessandria)(IT)**

(74) Representative: **Minoja, Fabrizio**
**Studio Consulenza Brevettuale Via Rossini, 8**
**I-20122 Milano(IT)**

(54) Chondroitin sulfate complexes having anti-inflammatory activity, processes for the preparation thereof and pharmaceutical compositions containing them.

(57) Copper-chondroitin sulfate complexes having anti-inflammatory, antiphlogistic and antioxidant activities, the process for the preparation thereof as well as pharmaceutical compositions containing them.

EP 0 450 398 A2

The present invention relates to a copper-chondroitinsulfuric acid complex, to a process for the preparation thereof and to pharmaceutical compositions containing it.

The use of copper salts or complexes in the treatment of patients suffering from acute or chronic arthritis dates back to the 40's and 50's, when it was an alternative to the use of gold salts, that are often poorly tolerated. However, the use of copper salts and complexes, particularly cuprolene, dicuprene and a copper-salicylic complex, was abandoned due to two basic reasons :

- discovery of hydrocortisone;
- lack of knowledge of the importance of copper in biological systems.

More recently, intra-articular administration of orgotein, also named superoxide dismutase, has been proposed. Superoxide dismutase is a copper-containing enzyme which can prevent superoxides, responsible for hyaluronic acid degradation in the synovial fluid, from being accumulated. Unfortunately, the efficacy of orgotein in the treatment of arthritis or infective diseases is severely restricted, since this substance has a 6 minute half-life.

Complexes of copper with amino acids (tryptophan, lysine), with non steroid anti-inflammatory drugs (indomethacin, ketoprofen, acetylsalicylic acid) or with fatty acids (oleic, lauric and caprylic acids) have been proposed, but the development thereof did not surpass the experimental and pre-clinical phases (Fourth International Conference on Superoxide and Superoxide Dismutase - N A Roberts, P A Robinson).

Chondroitinsulfuric acid, or chondroitin sulfate, belongs to the class of glycosaminoglycans, which are biological polymers abundant in soft connective and skeletal tissues, and it has been used for a long time as an hypolipemizing agent. European Patent N. 66,283 discloses "eustatic" compositions containing one non-toxic metal ion and a glycosaminoglycan selected from hyaluronic acid or chondroitin sulfate, useful as cicatrizants and generally to improve cell and tissue trophism. However, anti-inflammatory and anti-arthritis activities are not mentioned.

Finally, interactions between $Cu^{++}$ ion and chondroitin sulfate have already been studied from the chemicophysical point of view (e.g. in Inorg. Chim. Acta, 1983, 78(3)-129-34), but not from the biological and pharmacological one.

Now it has been found that copper-chondroitinsulfuric acid complexes have excellent pharmacological properties, particularly anti-inflammatory, anti-phlogistic, antioxidant and antiradical activities. Said copper-chondroitinsulfuric acid complexes can inhibit elastase, cathepsin, galactosidase and glucuronidase at micromolar concentrations.

The complexes of the invention can be obtained from sodium chondroitin sulfate or chondroitinsulfuric acid and a cupric salt such as $CuCl_2.2H_2O$ or $CuSO_4.5H_2O$ and they can contain from about 0.5% to about 10% Cu by weight. The starting chondroitin sulfate can have either low (1,000-3,000), medium (2,000-12,000) or high molecular weight.

The reaction of chondroitin sulfate with the cupric salt is carried out in an aqueous solution and the complex is precipitated by adding organic solvents, preferably $C_1$-$C_4$ alcohols.

The pharmacological properties of the complexes of the invention have been studied by means of the traditional experimental patterns for articular inflammation, such as the cotton pellet-induced granuloma and antigenic monoarticular arthritis, induced by ovoalbumin emulsified with Freund's adjuvant containing tuberculosis mycobacteria. Moreover, superoxide dismutase mimetic activity was evaluated by measuring the inhibition of cytochrome C reduction by the superoxide ion in the xanthine-xanthine oxidase enzyme reaction.

In all the considered tests, the compounds of the invention proved to be remarkably active, therefore they could be used in the therapeutical treatment of inflammatory diseases of various origin, such as degenerative arthropathies, rheumatoid arthritis, hip arthrosis, spondylarthrosis. For the envisaged therapeutic uses, the complexes of the invention will be administered in suitable pharmaceutical formulations for the oral or parenteral administrations (i.m., i.v. or intra-articular), such as capsules, tablets, syrups, vials and the like.

Daily dosages can vary within wide limits, thanks to the low toxicity of the complexes (i.p. $LD_{50}$ in the mouse: 675 mg/kg): generally they will range from 0.3 mg/kg/day to 3 mg/kg/day.

The following examples further illustrate the invention.

**EXAMPLE 1**

20 g of sodium chondroitin sulfate (MW 30,000±5,000) are dissolved in distilled water (400 ml) to a 5% concentration. The resulting solution is added with 2.5 g of $CuCl_2.2H_2O$ (equivalent to 0.636 g $Cu^{++}$) dissolved in 20 ml of water. The reaction mixture is stirred overnight, then treated with 95° ethanol (3 volumes) to obtain a precipitate which is pump filtered and washed with absolute ethanol. 20.7 g of a $Cu^{++}$-chondroitinsulfuric acid complex are obtained, containing 3% copper and 85% mucopolysaccharides. The product is in the form of a soft light blue powder, which is soluble in water, insoluble in organic solvents (ethanol, acetone, methanol). pH of a 1% aqueous solution is from 5 to 6.5.

By electrophoresis for 10 min at 450 Volts on Cellogel strips in 1M carbonate buffer, pH 11, an homogeneous band is evidenced which migrates to the anode. The band contains copper, which is quantitatively titrated by atomic absorption, and chondroitinsulfuric acid which is revealed by the light blue coloration obtained in the presence of a toluidine blue solution.

Studies on the Cu-chondroitinsulfuric acid bond were carried out subjecting a 2% aqueous solution of the complex to ultrafiltration through membranes of various cut-off (2,000-5,000-20,000-30,000) to confirm the stability of the complex.

## EXAMPLE 2

60 g of sodium chondroitin sulfate (MW 30,000±5,000) are dissolved in water (1,000 ml) to a 6% w/v concentration and transformed into the acidic form by passing them through a resin column Amberlite IR/120 (H$^+$). The obtained acid solution (pH 1.2) is slowly added with 10 ml of a 20% solution of $CuSO_4.5H_2O$ (equivalent to 0.508 g of Cu$^{++}$), under strong stirring. Stirring is continued for 4 hours, pH is adjusted to 6 with 1N NaOH, then the reaction mixture is treated with 95° ethanol to obtain a precipitate which is pump filtered and dried under vacuum in a dryer, to give 60.5 g of the complex, containing 0.75% Cu$^{++}$ and 85% mucopolysaccharides. The product is in the form of a light blue soft powder which is soluble in water, pH = 6. Chlorides : 2%; nitrogen : 2.5%; sulfur : 5.9%; proteins : 1.3%; humidity : 5.3%.

## EXAMPLE 3

A solution of 10 g of depolymerized sodium chondroitin sulfate (MW 3,000±1,000) in 150 ml of water is added with 40 ml of a $CuSO_4.5H_2O$ solution (equivalent to 84.7 mg of Cu$^{++}$). The mixture is stirred for 4 hours, then treated with 5 volumes of absolute ethanol to obtain a precipitate which is pump filtered and dried under vacuum in a dryer, to yield 10.5 g of a complex in the form of a hygroscopic light blue powder, which as a 4% aqueous solution is clear. The product has the following characteristics : copper : 0.75%; glucuronic acid : 25%; hexosamine : 26%; nitrogen : 3.0%; proteins : 2.0%; sulfur : 6%; chlorides : 2%.

## EXAMPLE 4

A solution of 10 g of depolymerized chondroitin sulfate (MW 12,000±2,000) in 150 ml of water is added with 40 ml of a $CuSO_4.5H_2O$ solution (equivalent to 84.7 mg Cu$^{++}$). After 4 hours under stirring, the mixture is precipitated with 5 volumes of ethanol, to obtain a precipitate which is decanted overnight, then centrifuged and washed 3 times with absolute ethanol, once with acetone, pump filtered and dried under vacuum in a dryer. 10.3 g of the complex are obtained, in the form of a hygroscopic light blue powder, having the following characteristics : copper : 0.84%; glucuronic acid : 25%; hexosamine : 26%; nitrogen : 3%; sulfur : 6%; proteins : 2%; chlorides : 2%.

## EXAMPLE 5

A solution of 50 g of sodium chondroitin sulfate (MW 30,000±5,000) in 750 ml of water is added with 20 g of $CuSO_4.5H_2O$ (equivalent to 5 g of Cu$^{++}$). The mixture is stirred overnight, then treated with 95° ethanol (3 volumes) to obtain a precipitate which is centrifuged and washed with absolute ethanol, to yield 56.2 g of a complex in the form of a light blue powder, having the following characteristics: soluble in water, pH = 4.5; copper : 7.37%; mucopolysaccharides : 83%; chlorides : 2%; sulfur : 5.8%; nitrogen : 2.9%; sodium : 6.20%.

## EXAMPLE 6

A solution of 50 g of sodium chondroitin sulfate (MW 30,000±5,000) in 500 ml of water is added with 30 g of $CuSO_4.5H_2O$ (equivalent to 7.63 g of Cu$^{++}$) dissolved in 200 ml of water. The mixture is stirred overnight, then treated with 95° ethanol (3 volumes) to obtain a precipitate which is centrifuged and washed with absolute ethanol, to yield 57.7 g of a complex containing 8.7% copper.

## EXAMPLE 7

20 g of sodium chondroitin sulfate (injectable grade, MW 30,000±5,000) are dissolved in distilled water to a 5% concentration. The obtained clear solution is added with 10 ml of a 20% solution of $CuSO_4.5H_2O$ (equivalent to 0.508 g Cu$^{++}$). The mixture is stirred for 12 hours, then it is treated with 95° ethanol to obtain a precipitate which is dried by pump filtration, dissolved in water (400 ml) and dialyzed during 24 hours against distilled water. By adding 3 volumes of 95° ethanol, 20.5 g of a complex are obtained, in the form of a soft light blue apyrogenic powder, which is soluble in water, having the following characteristics: copper : 0.75%; mucopolysaccharides : 98% (s.c.); humidity : 3.8%; sulfuric ashes : 20%; nitrogen : 2.6%; proteins : 0.79%; sulfur : 5.6%; free sulfates : less than 200 ppm; chlorides : 0.06%; sodium : 7.56%.

## Claims

1. Copper-chondroitin sulfate complexes.

2. Complexes as claimed in claim 1, containing from 0.5% to 10% cupric ion by weight.

3. Complexes as claimed in claims 1 or 2, in which chondroitin sulfate has low, medium or high molecular weight.

4. Copper-chondroitin sulfate complexes as therapeutic agents.

5. A process for the preparation of the complexes as claimed in claims 1 to 4, which comprises reacting chondroitin sulfate in the acid or salified form with the cupric ion in an aqueous solution, then precipitating the complex by addition of organic solvents.

6. Pharmaceutical compositions containing as the active ingredient a complex as claimed in claims 1 to 4, with a suitable carrier.

7. The use of the complexes as claimed in claims 1 to 4 for the preparation of a medicament having anti-inflammatory, antiphlogistic and antioxidant activities.

**Claims for the following Contracting States: ES and GR.**

1. A process for the preparation of copper-chondroitin sulfate complexes, which process comprises reacting chondroitin sulfate in the acid or salified form with the cupric ion in an aqueous solution, then precipitating the complex by addition of organic solvents.

2. A process as claimed in claim 1, wherein chondroitin-sulfate used has low, medium or high molecular weight.

3. A process for the preparation of copper-chondroitin sulfate complexes, wherein cupric ion is present in a concentration from 0.5 to 10% by weight.